# EUROPEAN PATENT APPLICATION

(11) **EP 4 745 178 A1**
(43) Date of publication of application: **20.05.2026**
(21) Application number: 24847894.3
(22) Date of filing: 27.06.2024
(51) Int. Cl.: C08G 69/48, C08G 69/02, C08G 69/16, A61K 47/34

(54) **ULTRA-HIGH MOLECULAR WEIGHT POLYGLUTAMIC ACID AND PREPARATION METHOD THEREFOR**

(30) Priority: 31.07.2023 CN 202310951089
(71) Applicant: Imeik Technology Development Co., Ltd., Beijing 100022 (CN)
(72) Inventor: GU, Shiwei, Beijing 100022 (CN); LI, Ruizhi, Beijing 100022 (CN); ZHANG, Kun, Beijing 100022 (CN)
(74) Representative: Curell Suñol S.L.P.
(86) International application number: PCT/CN2024/102006
(87) International publication number: WO 2025/025933

(57) **Abstract**

The present invention provides an ultra-high molecular weight Polyglutamic Acid (PGA) and a preparation method thereof. γ-protecting group-L-glutamate-N-carboxyanhydride (i.e., glutamate-N-carboxyanhydride) is used as a raw material, and PGA with a molecular weight of 1500-4000 kDa is prepared by using a metal hydride-promoted initiator-initiated polymerization technology. The present invention uses an anionic active ring-opening polymerization process, which is low in production cost and mild in reaction condition. The obtained PGA has a high molecular weight, and is applicable in the fields of drug delivery, tissue engineering materials, soft tissue repair, etc.

## Description

### Technical Field

The present invention belongs to the technical field of biomedical material preparation, and specifically, to an ultra-high molecular weight Polyglutamic Acid (PGA) and a preparation method thereof.

### Background

PGA is a polymer using glutamic acid as a structure unit, has a structure similar to that of polypeptide in an organism, and has good biocompatibility, biodegradability, and biosafety. Furthermore, since a PGA molecular chain has a large number of free carboxyl groups, in an aspect, PGA can not only be employed to modify bioactive molecules for drug delivery, but can also be cross-linked with multi-hydroxyl or multi-amino cross-linkers to form hydrogels for applications in tissue engineering, and in another aspect, the carboxyl group has the ability to bind water, causing the PGA to have high water absorption and moisture retention. Therefore, the PGA may be used in cosmetics as a moisturizing ingredient, etc., and become one of the most active biomedical polymers currently researched due to many advantages of the PGA.

Currently, there are three primary methods for synthesizing the PGA: an extraction method, a microbial fermentation method, and a chemical synthesis method. The extraction method includes extracting the PGA from natto. Due to the low PGA content in the natto, the extraction process is complex, production costs are high, and it is difficult to achieve large-scale production. The microbial fermentation method is the currently industrialized method for preparing the PGA with a molecular weight of 10 kDa to 2000 kDa. However, the method is complex in production process, hard in molecular weight control, and low in production capacity. The chemical synthesis method using γ-protecting group-L-glutamate-N-carboxyanhydride (i.e., glutamate NCA, same below) for ring-opening polymerization is an important synthesis method for kilogram-grade PGA, which is simple in process, easy to product purification, controllable in molecular weight of the obtained PGA, and small in distribution coefficient.

In the related art, in the chemical synthesis method, an alkaline reagent (e.g., triethylamine, sodium borohydride, sodium hydride, etc.) is often used as an initiator to prepare the PGA under inert gas protection. The preparation process is usually performed in a sealed glove box. For example, disclosed in patent CN102666566A is a method for, under argon protection, preparing a start PGA benzyl ester by using a glutamate monomer as a raw material and using triethylamine, sodium borohydride or a combination of the triethylamine and the sodium borohydride as the initiator, and then preparing a product PGA with a molecular weight of 12-72 kDa through hydrolysis. However, the product has a relatively low molecular weight, which is still significantly lower than that of products obtained through microbial fermentation. Furthermore, anhydrous and anaerobic environments need to be provided, leading to complex processes and strict reaction conditions, thus resulting in high preparation costs.

In the method for preparing the PGA through ring-opening polymerization, previous work has primarily focused on developing a new initiator to prepare high-molecular-weight polyglutamate (PBG), followed by deprotection of the ester groups to obtain the PGA with the corresponding molecular weight. Sodium hydride, potassium hydride, amine compounds such as primary amine and secondary amine, transition metal complexes, hexamethyldisilazane, trimethylsilyl derivatives, Al-Schiff base complexes, rare earth metal complexes, ammonium salt, amine borane Lewis acid-base pairs, etc. are developed successively. The use of these initiators, either individually or in combination, has significantly increased the molecular weight of the PGA. Patent US4161948A reports that triethylamine and sodium hydride are combined to initiate polymerization of PGA and leucine NCA monomers, so as to prepare a 25 kDa polymer. Patent CN101511862A also reports that potassium hydride, sodium hydride, cyclohexylamine, etc. are mixed to initiate preparation of an amino acid NCA copolymer, so as to obtain a copolymer with a molecular weight up to 25 kDa. Recently, Hua Lv et al. from Peking University "J Yuan, Z Yi, Z Li, Y Wang, & L Hua. (2018). A s-sn lewis pair-mediated ring-opening polymerization of α-amino acid n -carboxyanhydrides: fast kinetics, high molecular weight, and facile bioconjugation. ACS Macro Letters, 7,892-897." publicly reported preparation of a Poly-Gamma-Benzyl- L-Glutamate (PBLG) with a molecular weight up to 800 kDa using trimethyl tin-thiophenol as an initiator. However, the initiator thiophenol is a highly toxic compound, and the initiated PGA is unsuitable for biomedical applications. Furthermore, the molecular weight of the obtained product still differs from that of the product obtained through microbial fermentation.

Furthermore, a γ-protecting group-L-glutamate-N-carboxyanhydride (glutamate NCA) monomer is sensitive to water vapor, causing its synthesis to be performed mostly in a glove box or performed using a vacuum line mated with an experimental device. In order to solve this problem, Runhui Liu et al. from East China University of Science and Technology "Wu Y, Zhang D, Ma P, et al. Lithium hexamethyldisilazide initiated superfast ring opening polymerization of alpha-amino acid N-carboxyanhydrides[J]. Nature Communications, 2018, 9(1)." disclosed preparation of a PGA with a molecular weight of 280 kDa using lithium bis(trimethylsilyl)amide as an initiator to perform a reaction in an open system, so as to solve the problem of synthesis of a PGA under an air atmosphere. However, the obtained product is relatively low in molecular weight, which still largely differs from the molecular weight of the product obtained through microbial fermentation.

### Summary

In order to solve the above problem, the present invention provides a PGA with an ultra-high molecular weight that is prepared, in an air atmosphere, by using a hydride (e.g., magnesium hydride, calcium hydride, or copper hydride) corresponding to a metal with strong electronegativity and an initiator (e.g., triethylamine or pyridine) to form a complex initiation system, so as to initiate a ring-opening polymerization reaction.

A first aspect of the present invention provides an ultra-high molecular weight PGA, which is obtained through a ring-opening polymerization reaction that is initiated by a monomer and a complex initiation system, which is formed by a metal hydride and an initiator.

Further, the metal hydride is selected from one or more of magnesium hydride, calcium hydride, and copper hydride.

Further, the initiator is an organic amine compound and/or a heterocyclic compound.

Preferably, the organic amine compound is selected from one or more of triethylamine, dimethylamine, diethylamine, dipropylamine, diisopropylamine, N-ethylmethylamine, dibutylamine, diisobutylamine, di-sec-butylamine, di-tert-butylamine, diamylamine, dioctylamine, diallylamine, dicyclohexylamine, and diphenylamine.

Preferably, the heterocyclic compound is selected from one or more of pyridine, aziridine, pyrrole, pyrrolidine, imidazole, indole, piperidine, and purine.

In some embodiments of the present invention, the initiator is triethylamine.

In some embodiments of the present invention, the initiator is pyridine.

Further, in the polymerization reaction, a molar ratio of the monomer to the initiator is (1-50):1. In the complex initiation system, the content of the metal hydride is 1-30 wt% of the initiator, and specifically is, for example, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, or 30%, preferably 1%, 2%, 5%, 10%, 15%, 20%, 25%, or 30%.

Further, the monomer is glutamate-N-carboxyanhydride.

Further, the glutamate-N-carboxyanhydride is γ-alkyl-L-glutamate-N-carboxyanhydride or γ-aryl-L-glutamate-N-carboxyanhydride. The alkyl is selected from methyl, ethyl, tert-butyl, allyl, tert-butyl dimethyl, or the like. The aryl is selected from benzyl, or benzyl substituted with one or more substituents selected from halogen, phenolic hydroxyl, nitro, and a sulfo substituent, etc., benzhydryl, trityl, (ethylphenyl)methy, naphthylmethyl, anthrylmethyl, or the like. The above groups may all be used as carboxyl protecting groups at a γ position of glutamic acid.

In some embodiments of the present invention, the glutamate-N-carboxyanhydride is γ -benzyl-L-glutamate-N-carboxyanhydride, γ-tert-butyl-L-glutamate acid-N-carboxyanhydride, or γ-ethyl-L-glutamate acid-N-carboxyanhydride.

Further, a molecular weight of the ultra-high molecular weight PGA is 1500-4000 kDa, with a molecular weight distribution coefficient 1<PD<2.

A second aspect of the present invention provides a method for preparing an ultra-high molecular weight PGA. The preparation method includes the following steps.
(1) Synthesis of polyglutamate: a glutamate-N-carboxyanhydride monomer (glutamate NCA), an organic solvent A, a metal hydride, and an initiator are mixed to perform a reaction, so as to obtain the polyglutamate.
(2) Synthesis of PGA: the polyglutamate obtained in step (1), an organic solvent B, and a deprotection agent are mixed to perform a reaction, so as to obtain an ultra-high molecular weight PGA.

The molecular weight of the ultra-high molecular weight PGA is 1500-4000 kDa, and specifically is, for example, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, or 4000 kDa.

Further, the glutamate-N-carboxyanhydride monomer is γ-alkyl-L-glutamate-N-carboxyanhydride or γ-aryl-L-glutamate-N-carboxyanhydride.

Preferably, the alkyl is selected from methyl, ethyl, tert-butyl, allyl, or tert-butyl dimethyl; and the aryl is selected from benzyl, benzyl substituted with one or more substituents selected from halogen, phenolic hydroxyl, nitro, and a sulfo substituent, benzhydryl, trityl, (ethylphenyl)methy, naphthylmethyl, or anthrylmethyl.

More preferably, the glutamate-N-carboxyanhydride monomer is γ -benzyl-L-glutamate-N-carboxyanhydride, γ-tert-butyl-L-glutamate-N-carboxyanhydride, or γ-ethyl-L-glutamate-N-carboxyanhydride.

It is found in the present invention that, the metal hydride is selective; during ring-opening polymerization, a metal and a monomer anion (i.e., glutamate NCA anion) form ion pairs in the form of M-N and M-O bonds; if the electronegativity of the metal in the metal hydride is stronger, for example, calcium (1.00), magnesium (1.31), or copper (1.90) in the present invention, the formed ionic bond is weaker in bond energy and further exhibits more covalent character; and a currently formed ion pair is a loose ion pair, which is more stable, has good solubility in a reaction system, and can better promote the initiator to perform a chain propagation process, such that the PGA with an ultra-high molecular weight may be prepared. A loose ion pair chain initiation and propagation process is shown in a formula (I).

If the electronegativity of the metal is weaker, an ionic bond is more easily formed. The formed ion pair is a tight ion pair. The ion pair is poor in solubility in the system, metal ions strongly interact with monomer anions, forming intimate ion-pairs, not facilitating the proceeding of the chain propagation process. For example, the polymer that is reported in the patents US4161948A and CN101511862A mentioned in the Background and obtained by, individually or in combination, using the sodium hydride or potassium hydride with the initiator triethylamine has a relatively low molecular weight, which is only up to 25 kDa. This may be attributed to the use of metals sodium (0.93) and potassium (0.82) with low electronegativity, and a intimate ion-pair is formed, not facilitating chain propagation, such that the prepared PGA is small in degree of polymerization, and low in molecular weight. A intimate ion-pair chain initiation and propagation process is shown in a formula (II).

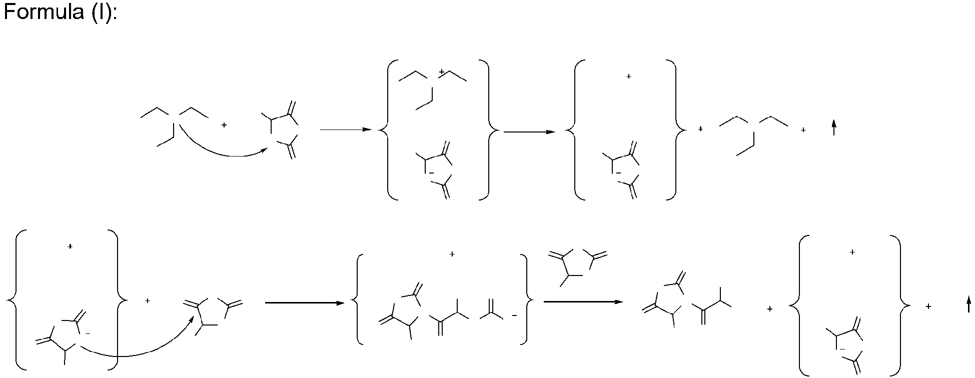

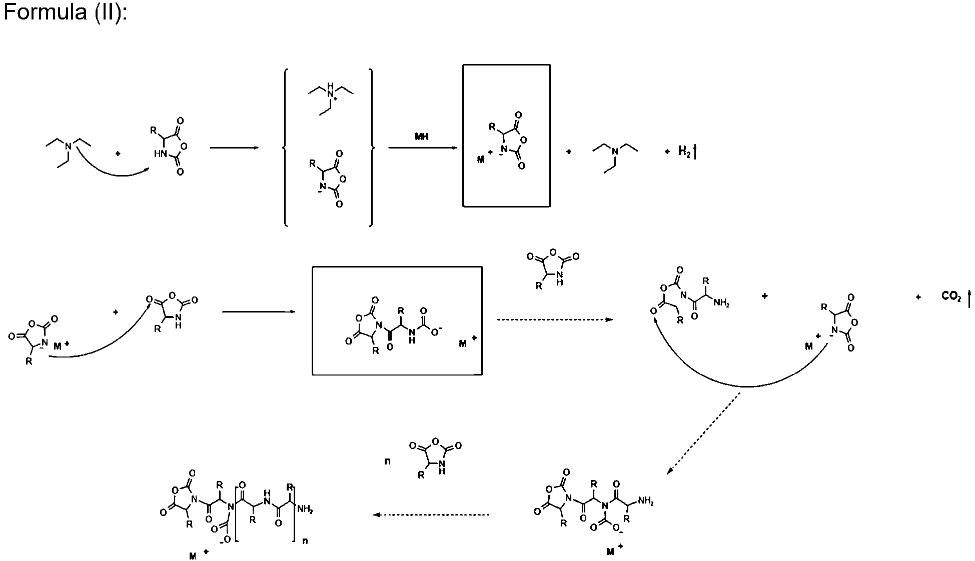

Further, in step (1), the glutamate-N-carboxyanhydride monomer is first dissolved in the organic solvent A, and a premix of the metal hydride and the initiator are then added to mix and react, so as to obtain the polyglutamate.

In step (2), the polyglutamate obtained in step (1) is first dissolved in the organic solvent B, and the deprotection agent is then added to mix and react, so as to obtain the ultra-high molecular weight PGA.

Further, in step (1), the organic solvent A is selected from one or more of 1,4-dioxane, hexane, cyclohexane, dichloromethane, chloroform, tetrahydrofuran, N,N-dimethylformamide, dimethyl sulfoxide, diethyl ether, diisopropyl ether, acetonitrile, methanol, ethanol, ethyl acetate, benzene, and toluene, preferably, 1,4-dioxane.

Further, in step (1), the metal hydride is selected from one or more of magnesium hydride, calcium hydride, and copper hydride.

Further, in step (1), the initiator is an organic amine compound and/or a heterocyclic compound.

Preferably, the organic amine compound is selected from one or more of triethylamine, dimethylamine, diethylamine, dipropylamine, diisopropylamine, N-ethylmethylamine, dibutylamine, diisobutylamine, di-sec-butylamine, di-tert-butylamine, diamylamine, dioctylamine, diallylamine, dicyclohexylamine, and diphenylamine.

Preferably, the heterocyclic compound is selected from one or more of pyridine, aziridine, pyrrole, pyrrolidine, imidazole, indole, piperidine, and purine.

In some embodiments of the present invention, the initiator is triethylamine.

In some embodiments of the present invention, the initiator is pyridine.

Further, in step (1), a molar ratio of the glutamate-N-carboxyanhydride monomer to the initiator is (1-50):1, and is specifically, for example, 1:1, 5:1, 10:1, 15:1, 20:1, 25:1, 30:1, 35:1, 40:1, 45:1, or 50:1, preferably (5-20):1.

Further, in step (1), the content of the metal hydride is 1-30 wt% of the initiator, and specifically is, for example, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, or 30%, preferably 1%, 2%, 5%, 10%, 15%, 20%, 25%, or 30%.

Further, in step (1), the reaction is performed at room temperature, which is specifically, for example, 20, 21, 22, 23, 24, 25, 26, 27, 28, 29, or 30 °C; and a reaction time is 1-5 days, and is specifically, for example, 1, 2, 3, 4, or 5 days.

Further, step (1) further includes steps of performing alcohol precipitation and drying on the polyglutamate.

Further, a solvent used for alcohol precipitation is ethanol.

Further, in step (2), the organic solvent B is selected from one or more of dichloromethane, chloroform, tetrahydrofuran, N,N-dimethylformamide, dimethyl sulfoxide, 1,4-dioxane, hexane, cyclohexane, diethyl ether, diisopropyl ether, acetonitrile, methanol, ethanol, ethyl acetate, benzene, and toluene, preferably, dichloromethane.

Further, in step (2), the deprotection agent is a silane protective agent, and is selected from one or more of iodotrimethylsilane, tert-butyldimethylsilyl chloride, triisopropylsilyl chloride, trifluoromethanesulfonic acid tert-butyldimethylsilyl ester, and triisopropylsilyl trifluoromethanesulfonate, preferably iodotrimethylsilane.

Further, in step (2), a molar ratio of the polyglutamate to the deprotection agent is 1:(1-3), and is specifically, for example, 1:1, 1:1.2, 1:1.4, 1:1.6, 1:1.8, 1:2, 1:2.5, or 1:3, preferably 1:(1-2.5), and more preferably 1:1.6.

Further, in step (2), the reaction is performed at room temperature, which is specifically, for example, 20, 21, 22, 23, 24, 25, 26, 27, 28, 29, or 30 °C; and a reaction time is 12-24 h, and is specifically, for example, 12, 13, 14, 15, 16, 17, 18, 19, 20, 21, 22, 23, or 24 h.

Further, step (2) further includes steps of performing hydrolysis, layering, washing, system pH adjustment, alcohol precipitation, re-dissolution, dialysis, and freeze-drying on the polyglutamate.

Further, a solvent used for dialysis is water.

Further, a detergent used for washing is dichloromethane.

Further, the pH value of the system is adjusted to 7-9, and specifically is, for example, 7, 7.2, 7.4, 7.6, 7.8, 8, 8.2, 8.4, 8.6, 8.8, or 9.

Further, a solvent used for alcohol precipitation is ethanol.

Further, a solvent used for re-dissolution is water.

The technical principle of the present invention is as follows (in an example of using the initiator as triethylamine and using a protective agent as iodotrimethylsilane).

Polymerization mechanism of polyglutamate solution:

Chain initiation:

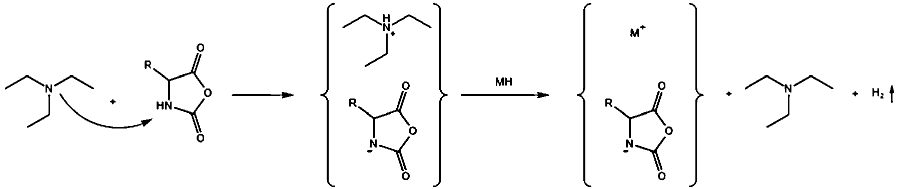

Chain propagation and termination:

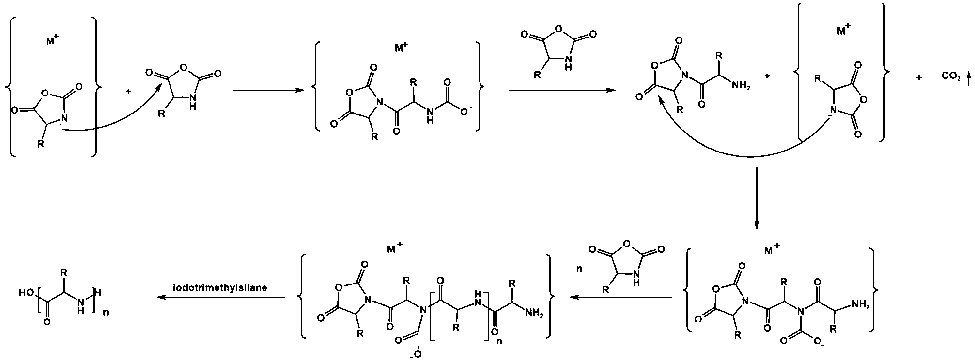

The triethylamine is used as an alkaline initiator; protons of the glutamate-N-carboxyanhydride monomer amide are first captured to form an initiator-derived active ion pair; then, the ion pair reacts with the system solid-phase metal hydride at an interface to generate a metal ion-monomer active intermediate ion pair; and the ion pair continues to attack an NCA monomer to form a dimer. A glutamate NCA anion in the dimer forms a loose ion pair with the metal ion. With regard to the presence of the loose ion pair, in an aspect, a glutamate NCA active intermediate is stabilized to avoid the occurrence of side reactions; and in another aspect, the loose ion pair has good solubility in the organic solvent, the metal ion exerts weak interactions with the glutamate NCA anion, facilitating the attacking of a new monomer by the glutamate NCA anion end so as to form the monomer active intermediate, thereby promoting chain propagation. Therefore, the polyglutamate with an ultra-high molecular weight may be prepared.

In an embodiment of the present invention, a step of synthesizing a PGA benzyl ester includes: a Schlenk tube is washed with chromic acid, and end sealing is performed with chlorotrimethylsilane; γ-benzyl-L-glutamate-N-carboxyanhydride is first added; then 1,4-dioxane is added; triethylamine containing a metal hydride is added; after well mixing through shaking, a reaction is performed for 3 days at room temperature; then the obtained viscous solution is diluted with 1,4-dioxane, and then dropwise added to ethanol to induce precipitation; and the PGA benzyl ester may be obtained by drying the precipitate.

In an embodiment of the present invention, a step of synthesizing a PGA includes: the obtained PGA benzyl ester is added to dichloromethane; then iodotrimethylsilane is added and stirred overnight at room temperature, where a molar ratio of the iodotrimethylsilane to the Poly-Gamma-Benzyl- L-Glutamate (PBLG) is 1.6:1; water is added on the next day and stirred to hydrolyze trimethylsilyl polyglutamate, and a lower layer is separated to remove impurities; the mixture is washed several times with dichloromethane to remove residual impurities; a starch potassium iodide solution is used to test whether iodine in the mixture is completely removed; the pH of the mixture is adjusted to 7-9; then precipitation is performed by using 5-fold the volume of ethanol, and the obtained precipitate is redissolved in purified water; salts are removed by dialysis over three days; and then the PGA is obtained by freeze drying.

In another embodiment of the present invention, a step of synthesizing a Poly-Gamma-tert-butyl-L-Glutamate includes: a Schlenk tube is washed with chromic acid, and the tube is sealed with chlorotrimethylsilane; γ-tert-butyl-L-glutamate-N-carboxyanhydride is first added; then 1,4-dioxane is added; pyridine containing a metal hydride is added; after well mixing through shaking, a reaction is performed for 3 days at room temperature; then the obtained viscous solution is diluted with 1,4-dioxane, and then dropwise added to ethanol to induce precipitation; and the Poly-Gamma-tert-butyl-L-Glutamate is obtained by drying the precipitate.

In another embodiment of the present invention, the step of synthesizing the PGA includes: the obtained Poly-Gamma-tert-butyl-L-Glutamate is added to dichloromethane; then iodotrimethylsilane is added and stirred overnight at room temperature, where a molar ratio of the iodotrimethylsilane to the Poly-Gamma-tert-butyl-L-Glutamate is 2.8:1; water is added on the next day and stirred to hydrolyze Poly-Gamma-Trimethylsilyl-L-Glutamate, and a lower layer is separated to remove impurities; the mixture is washed several times with dichloromethane to remove residual impurities; a starch potassium iodide solution is used to test whether iodine in the mixture is completely removed; the pH of the mixture is adjusted to 7-9; then precipitation is performed by using 5-fold the volume of ethanol, and the obtained precipitate is redissolved in purified water; salts are removed by dialysis over three days; and then the PGA is obtained by freeze drying.

A third aspect of the present invention provides applications of the ultra-high molecular weight PGA of the first aspect or an ultra-high molecular weight PGA prepared by the preparation method of the second aspect in drug delivery, tissue engineering material preparation, and soft tissue repair.

Compared with the related art, the present invention has the following prominent characteristics.
(1) In the present invention, magnesium hydride, calcium hydride, and copper hydride are specifically selected to form the complex initiation system with the initiator (e.g., organic amine or heterocyclic compound), and the PGA is prepared through Anionic Active Ring-opening Polymerization (AARP). During ring-opening polymerization, the metal and the monomer anion form the ion pairs in the form of M-N and M-O bonds. By using the characteristics of strong electronegativity of the metal in the metal hydride, the loose ion pair of the metal ion-monomer active intermediate may be formed with the monomer active intermediate. In an aspect, a glutamate NCA active intermediate is stabilized to avoid the occurrence of side reactions; and in another aspect, the loose ion pair has good solubility in the organic solvent, the metal ion exerts weak interactions with the polyglutamate NCA anion, facilitating the attacking of a new monomer by the glutamate NCA anion end so as to form the monomer active intermediate, thereby promoting chain propagation. Therefore, the polyglutamate with an ultra-high molecular weight can be prepared.
(2) The metal hydride used in the present invention promotes the stable presence of the loose ion pair intermediate formed by the initiation system in a non-polar solvent. After the monomer in the system completely reacts, the monomer is continuously added without terminating chain growth, a chain growth may be further performed to expand a molecular weight. The AARP of the present invention enables continuous and highly stable promotion of chain growth and chain propagation. The molecular weight of the PGA prepared may reach 1500-4000 kDa, thereby providing greater flexibility in PGA applications.
(3) In the present invention, the metal hydride is used to promote the complex initiation system initiated by the initiator. The reaction may be performed in an open system at room temperature and pressure. The PGA with an ultra-high molecular weight (1500-4000 kDa) can be obtained under synthesis conditions without a glove box, and the molecular weight is much higher than that (30-280 kDa) of PGA synthesized by a ring-opening polymerization technology currently using a glove box. Therefore, the synthesis process of the PGA of the present invention is performed without using the glove box, and is mild in reaction conditions, thereby greatly reducing production costs.
(4) The PGA prepared in the present invention has the molecular weight higher to that of the PGA produced by traditional microbial fermentation, and is controllable in molecular weight and small in molecular distribution coefficient. Furthermore, compared to microbial fermentation, the process method of the present invention is simple in operation, and the product is easy to purify.
(5) The ultra-high molecular weight PGA synthesized in the present invention has wide applications, and may be used in the fields such as drug delivery, tissue engineering materials, soft tissue repair, etc.

### Brief Description of the Drawings

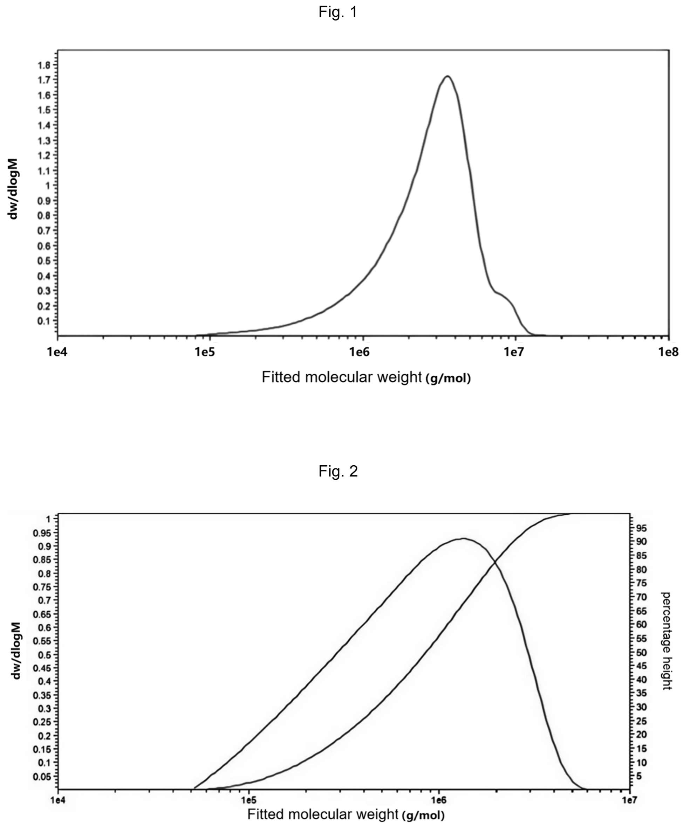

Fig. 1 is a molecular weight diagram of a GPC test of Poly-γ-Benzyl-L-Glutamate prepared in Embodiment 4 of the present invention.
Fig. 2 is a molecular weight diagram of a GPC test of poly-L-glutamic acid prepared in Embodiment 4 of the present invention.

### Detailed Description of the Embodiments

Unless otherwise defined, all scientific and technical terms used in the present invention have the same meaning as is commonly understood by those skilled in the art to which the present invention relates.

In the present invention, the term "room temperature" refers to the temperature of an object being close to or identical to the temperature of a space (e.g., a space such as a fume hood where the object is located). Generally, room temperature is approximately 20 °C to approximately 30 °C, or approximately 22 °C to approximately 27 °C, or approximately 25 °C.

In the present invention, the term "air atmosphere" refers to a mixture of gases that surrounds the Earth, primarily including oxygen, carbon dioxide, nitrogen, rare gases, and other substances (e.g., water vapor, etc.).

In the present invention, the term "PD" refers to a polydispersity index that describes the molecular weight distribution of polymers. The PD is a quantitative indicator of molecular weight distribution in the polymers, and is specifically calculated by dividing a weight-average molecular weight (Mw) of the polymer by a number-average molecular weight (Mn). If the value of the PD is smaller, it indicates that the molecular weight distribution in the polymers is narrower, that is, the difference between the molecular weights in the polymers is smaller; and on the contrary, if the value of the PD is larger, it indicates that the molecular weight distribution in the polymers is wider, that is, the difference between the molecular weights in the polymers is larger. In general, the value of the PD ranges from 1 to 10, and the PD of a good polymer material is generally between 1 and 2.

Main raw materials such as the metal hydride, triethylamine, γ -benzyl-L-glutamate-N-carboxyanhydride, γ-tert-butyl-L-glutamate-N-carboxyanhydride, γ-ethyl-L-glutamate-N-carboxyanhydride, 1,4-dioxane, etc. used herein are all commercially available products.

The contents of various publications, patents, and disclosed patent specifications cited herein are incorporated by reference in their entirety.

The technical solutions of the present invention will be clearly and completely described below with reference to the embodiments of the present invention. It is apparent that the described embodiments are merely part of the embodiments of the present invention, not all the embodiments. Based on the embodiments in the present invention, all other embodiments obtained by those of ordinary skill in the art without creative work shall fall within the protection scope of the present invention.

### Embodiment 1: 1% magnesium hydride promoting preparation of poly-L-glutamic acid 1 using triethylamine

### 1. Preparation of Poly-γ-Benzyl-L-Glutamate 1

2.0 g of γ-benzyl-L-glutamate-N-carboxyanhydride was added, in an air atmosphere, to a Schlenk tube that was washed with chromic acid and had an end sealed with chlorotrimethylsilane; then 50 ml of ultra-dry 1,4-dioxane and a complex initiation system of ultra-dry triethylamine and magnesium hydride (adding the magnesium hydride to the triethylamine to form a premix), where the amount of the triethylamine added was 0.1537 g, and the amount of the magnesium hydride added was 1% of the mass of the triethylamine; a molar ratio of a monomer to the initiator triethylamine was 5:1; and then the mixture was well-mixed and allowed to react for three days at room temperature. After three days, the solution was viscous, was diluted with 1,4-dioxane, and then dropwise added to absolute ethanol to induce precipitation; and after the precipitate was filtered under vacuum and dried for 24 h, the Poly-γ-Benzyl-L-Glutamate 1 was obtained.

### 2. Preparation of poly-L-glutamic acid 1

The obtained Poly- γ -Benzyl-L-Glutamate 1 was added to dichloromethane; then iodotrimethylsilane was added and stirred overnight at room temperature, where a molar ratio of the Poly-γ-Benzyl-L-Glutamate to the iodotrimethylsilane was 1:1.6; then water was added on the next day and stirred to hydrolyze poly-γ-trimethylsilyl-glutamate, and a lower layer was then separated to remove impurities; the mixture was washed several times with dichloromethane to remove residual impurities; a starch potassium iodide solution was used to test whether iodine in the mixture was completely removed; the pH of the mixture was adjusted to 7-9; then precipitation was performed by using 5-fold the volume of ethanol, and the obtained precipitate was re-dissolved with purified water; salts were removed by dialysis over three days; and then the poly-L-glutamic acid 1 was obtained through freeze drying.

### Embodiment 2: 5% magnesium hydride promoting preparation of poly-L-glutamic acid 2 using triethylamine

### 1. Preparation of Poly-γ-Benzyl-L-Glutamate 2

2.0 g of γ-benzyl-L-glutamate-N-carboxyanhydride was added, in an air atmosphere, to a Schlenk tube that was washed with chromic acid and had an end sealed with chlorotrimethylsilane; then 50 ml of ultra-dry 1,4-dioxane and a complex initiation system of ultra-dry triethylamine and magnesium hydride (adding the magnesium hydride to the triethylamine to form a premix), where the amount of the triethylamine added was 0.1537 g, and the amount of the magnesium hydride added was 5% of the mass of the triethylamine; a molar ratio of a monomer to the initiator triethylamine was 5:1; and then the mixture was well-mixed and allowed to react for three days at room temperature. After three days, the solution was viscous, was diluted with 1,4-dioxane, and then dropwise added to absolute ethanol to induce precipitation; and after the precipitate was filtered under vacuum and dried for 24 h, the Poly-γ-Benzyl-L-Glutamate 2 was obtained.

### 2. Preparation of poly-L-glutamic acid 2

An operation was the same as S2 in Embodiment 1, and the poly-L-glutamic acid 2 was ultimately obtained.

### Embodiment 3: 10% magnesium hydride promoting preparation of poly-L-glutamic acid 3 using triethylamine

### 1. Preparation of Poly-γ-Benzyl-L-Glutamate 3

2.0 g of γ-benzyl-L-glutamate-N-carboxyanhydride was added, in an air atmosphere, to a Schlenk tube that was washed with chromic acid and had an end sealed with chlorotrimethylsilane; then 50 ml of ultra-dry 1,4-dioxane and a complex initiation system of ultra-dry triethylamine and magnesium hydride (adding the magnesium hydride to the triethylamine to form a premix), where the amount of the triethylamine added was 0.1537 g, and the amount of the magnesium hydride added was 10% of the mass of the triethylamine; a molar ratio of a monomer to the initiator triethylamine was 5:1; and then the mixture was well-mixed and allowed to react for three days at room temperature. After three days, the solution was viscous, was diluted with 1,4-dioxane, and then dropwise added to absolute ethanol to induce precipitation; and after the precipitate was filtered under vacuum and dried for 24 h, the Poly-γ-Benzyl-L-Glutamate 3 was obtained.

### 2. Preparation of poly-L-glutamic acid 3

An operation was the same as S2 in Embodiment 1, and the poly-L-glutamic acid 3 was ultimately obtained.

### Embodiment 4: 20% magnesium hydride promoting preparation of poly-L-glutamic acid 4 using triethylamine

### 1. Preparation of Poly-γ-Benzyl-L-Glutamate 4

2.0 g of γ-benzyl-L-glutamate-N-carboxyanhydride was added, in an air atmosphere, to a Schlenk tube that was washed with chromic acid and had an end sealed with chlorotrimethylsilane; then 50 ml of ultra-dry 1,4-dioxane and a complex initiation system of ultra-dry triethylamine and magnesium hydride (adding the magnesium hydride to the triethylamine to form a premix), where the amount of the triethylamine added was 0.1537 g, and the amount of the magnesium hydride added was 20% of the mass of the triethylamine; a molar ratio of a monomer to the initiator triethylamine was 5:1; and then the mixture was well-mixed and allowed to react for three days at room temperature. After three days, the solution was viscous, was diluted with 1,4-dioxane, and then dropwise added to absolute ethanol to induce precipitation; and after the precipitate was filtered under vacuum and dried for 24 h, the Poly-γ-Benzyl-L-Glutamate 4 was obtained.

### 2. Preparation of poly-L-glutamic acid 4

An operation was the same as S2 in Embodiment 1, and the poly-L-glutamic acid 4 was ultimately obtained.

### Embodiment 5: 30% magnesium hydride promoting preparation of poly-L-glutamic acid 5 using triethylamine

### 1. Preparation of Poly-γ-Benzyl-L-Glutamate 5

2.0 g of γ-benzyl-L-glutamate-N-carboxyanhydride was added, in an air atmosphere, to a Schlenk tube that was washed with chromic acid and had an end sealed with chlorotrimethylsilane; then 50 ml of ultra-dry 1,4-dioxane and a complex initiation system of ultra-dry triethylamine and magnesium hydride (adding the magnesium hydride to the triethylamine to form a premix), where the amount of the triethylamine added was 0.1537 g, and the amount of the magnesium hydride added was 30% of the mass of the triethylamine; a molar ratio of a monomer to the initiator triethylamine was 5:1; and then the mixture was well-mixed and allowed to react for three days at room temperature. After three days, the solution was viscous, was diluted with 1,4-dioxane, and then dropwise added to absolute ethanol to induce precipitation; and after the precipitate was filtered under vacuum and dried for 24 h, the Poly-γ-Benzyl-L-Glutamate 5 was obtained.

### 2. Preparation of poly-L-glutamic acid 5

An operation was the same as S2 in Embodiment 1, and the poly-L-glutamic acid 5 was ultimately obtained.

### Embodiment 6: 2% calcium hydride promoting preparation of poly-L-glutamic acid 6 using triethylamine

### 1. Preparation of Poly-γ-Benzyl-L-Glutamate 6

2.0 g of γ-benzyl-L-glutamate-N-carboxyanhydride was added, in an air atmosphere, to a Schlenk tube that was washed with chromic acid and had an end sealed with chlorotrimethylsilane; then 50 ml of ultra-dry 1,4-dioxane and a complex initiation system of ultra-dry triethylamine and calcium hydride (adding the calcium hydride to the triethylamine to form a premix), where the amount of the triethylamine added was 0.1537 g, and the amount of the calcium hydride added was 2% of the mass of the triethylamine; a molar ratio of a monomer to the initiator triethylamine was 5:1; and then the mixture was well-mixed and allowed to react for three days at room temperature. After three days, the solution was viscous, was diluted with 1,4-dioxane, and then dropwise added to absolute ethanol to induce precipitation; and after the precipitate was filtered under vacuum and dried for 24 h, the Poly-γ-Benzyl-L-Glutamate 6 was obtained.

### 2. Preparation of poly-L-glutamic acid 6

An operation was the same as S2 in Embodiment 1, and the poly-L-glutamic acid 6 was ultimately obtained.

### Embodiment 7: 2% copper hydride promoting preparation of poly-L-glutamic acid 7 using triethylamine

### 1. Preparation of Poly-γ-Benzyl-L-Glutamate 7

2.0 g of γ-benzyl-L-glutamate-N-carboxyanhydride was added, in an air atmosphere, to a Schlenk tube that was washed with chromic acid and had an end sealed with chlorotrimethylsilane; then 50 ml of ultra-dry 1,4-dioxane and a complex initiation system of ultra-dry triethylamine and copper hydride (adding the copper hydride to the triethylamine to form a premix), where the amount of the triethylamine added was 0.1537 g, and the amount of the copper hydride added was 2% of the mass of the triethylamine; a molar ratio of a monomer to the initiator triethylamine was 5:1; and then the mixture was well-mixed and allowed to react for one day at room temperature. After one day, the solution was viscous, was diluted with 1,4-dioxane, and then dropwise added to absolute ethanol to induce precipitation; and after the precipitate was filtered under vacuum and dried for 24 h, the Poly-γ-Benzyl-L-Glutamate 7 was obtained.

### 2. Preparation of poly-L-glutamic acid 7

An operation was the same as S2 in Embodiment 1, and the poly-L-glutamic acid 7 was ultimately obtained.

### Embodiment 8: 25% calcium hydride promoting preparation of poly-L-glutamic acid 8 using pyridine

### 1. Preparation of poly-γ-tert-butyl-L-glutammate

2.0 g of γ-tert-butyl-L-glutamate-N-carboxyanhydride was added, in an air atmosphere, to a Schlenk tube that was washed with chromic acid and had an end sealed with chlorotrimethylsilane; then 50 ml of ultra-dry 1,4-dioxane and a complex initiation system of ultra-dry pyridine and calcium hydride (adding the calcium hydride to the pyridine to form a premix), where the amount of the pyridine added was 0.1381 g, and the amount of the calcium hydride added was 25% of the mass of the pyridine; a molar ratio of a monomer to the initiator pyridine was 5:1; and then the mixture was well-mixed and allowed to react for three days at room temperature. After three days, the solution was viscous, was diluted with 1,4-dioxane, and then dropwise added to absolute ethanol to induce precipitation; and after the precipitate was filtered under vacuum and dried for 24 h, the poly-γ-tert-butyl-L-glutamate was obtained.

### 2. Preparation of poly-L-glutamic acid 8

The obtained poly-γ-tert-butyl-L-glutamate was added to dichloromethane; then iodotrimethylsilane was added and stirred overnight at room temperature, where a molar ratio of the Poly-γ-tert-butyl-L-glutamate to the iodotrimethylsilane was 1:2.8; then water was added on the next day and stirred to hydrolyze poly-γ-trimethylsilyl-L-glutamate, and a lower layer was then separated to remove impurities; the mixture was washed several times with dichloromethane to remove residual impurities; a starch potassium iodide solution was used to test whether iodine in the mixture was completely removed; the pH of the mixture was adjusted to 7-9; then precipitation was performed by using 5-fold the volume of ethanol, and the obtained precipitate was redissolved in purified water; salts were removed by dialysis over three days; and then the poly-L-glutamic acid 8 was obtained through freeze drying.

### Embodiment 9: 2% calcium hydride promoting preparation of poly-L-glutamic acid 9 using triethylamine, secondary monomer addition

### 1. Preparation of Poly-γ-Benzyl-L-Glutamate 8

2.0 g of γ-benzyl-L-glutamate-N-carboxyanhydride was added, in an air atmosphere, to a Schlenk tube that was washed with chromic acid and had an end sealed with chlorotrimethylsilane; then 50 ml of ultra-dry 1,4-dioxane and a complex initiation system of ultra-dry triethylamine and calcium hydride (adding the calcium hydride to the triethylamine to form a premix), where the amount of the triethylamine added was 0.1537 g, and the amount of the calcium hydride added was 2% of the mass of the triethylamine; a molar ratio of a monomer to the initiator triethylamine was 5:1; and then the mixture was well-mixed and allowed to react for three days at room temperature. After three days, the solution was viscous, at the moment, the reaction was not terminated, and 2.0 g of a γ-benzyl-L-glutamate-N-carboxyanhydride monomer was dissolved in the ultra-dry 1,4-dioxane and then added to a reaction system; then the reaction was performed for two more days, at the moment, the obtained produce was diluted with 1,4-dioxane, and then dropwise added to absolute ethanol to induce precipitation; and after the precipitate was filtered under vacuum and dried for 24 h, the Poly-γ-Benzyl-L-Glutamate 8 was obtained.

### 2. Preparation of poly-L-glutamic acid 9

An operation was the same as S2 in Embodiment 1, and the poly-L-glutamic acid 9 was ultimately obtained.

### Embodiment 10: 10% magnesium hydride promoting preparation of poly-L-glutamic acid 10 using triethylamine

### 1. Preparation of poly-γ-ethyl-L-glutamate

2.0 g of γ-ethyl-L-glutamate-N-carboxyanhydride was added, in an air atmosphere, to a Schlenk tube that was washed with chromic acid and had an end sealed with chlorotrimethylsilane; then 50 ml of ultra-dry 1,4-dioxane and a complex initiation system of ultra-dry triethylamine and magnesium hydride (adding the magnesium hydride to the triethylamine to form a premix), where the amount of the triethylamine added was 0.2008 g, and the amount of the magnesium hydride added was 10% of the mass of the triethylamine; a molar ratio of a monomer to the initiator triethylamine was 20:1; and then the mixture was well-mixed and allowed to react for three days at room temperature. After three days, the solution was viscous, was diluted with 1,4-dioxane, and then dropwise added to absolute ethanol to induce precipitation; and after the precipitate was filtered under vacuum and dried for 24 h, the poly-γ-ethyl-L-glutamate was obtained.

### 2. Preparation of poly-L-glutamic acid 10

The obtained poly-γ-ethyl-L-glutamate was added to dichloromethane; then iodotrimethylsilane was added and stirred overnight at room temperature, where a molar ratio of the poly-γ-ethyl-L-glutamate to the iodotrimethylsilane was 1:1.6; then water was added on the next day and stirred to hydrolyze trimethylsilyl polyglutamate, and a lower layer was then separated to remove impurities; the mixture was washed several times with dichloromethane to remove residual impurities; a starch potassium iodide solution was used to test whether iodine in the mixture was completely removed; the pH of the mixture was adjusted to 7-9; then precipitation was performed by using 5-fold the volume of ethanol, and the obtained precipitate was redissolved in purified water; salts were removed by dialysis over three days; and then the poly-L-glutamic acid 10 was obtained through freeze drying.

### Comparative example 1: 35% magnesium hydride promoting preparation of poly-L-glutamic acid 11 using triethylamine

### 1. Preparation of Poly-γ-Benzyl-L-Glutamate 9

2.0 g of γ-benzyl-L-glutamate-N-carboxyanhydride was added, in an air atmosphere, to a Schlenk tube that was washed with chromic acid and had an end sealed with chlorotrimethylsilane; then 50 ml of ultra-dry 1,4-dioxane and a complex initiation system of ultra-dry triethylamine and magnesium hydride (adding the magnesium hydride to the triethylamine to form a premix), where the amount of the triethylamine added was 0.1537 g, and the amount of the magnesium hydride added was 35% of the mass of the triethylamine; a molar ratio of a monomer to the initiator triethylamine was 5:1; and then the mixture was well-mixed and allowed to react for three days at room temperature. After three days, the solution was viscous, was diluted with 1,4-dioxane, and then dropwise added to absolute ethanol to induce precipitation; and after the precipitate was filtered under vacuum and dried for 24 h, the Poly-γ-Benzyl-L-Glutamate 9 was obtained.

### 2. Preparation of poly-L-glutamic acid 11

An operation was the same as S2 in Embodiment 1, and the poly-L-glutamic acid 11 was ultimately obtained.

### Comparative example 2: Preparation of poly-L-glutamic acid 12 through triethylamine catalysis

### 1. Preparation of Poly-γ-Benzyl-L-Glutamate 10

2.0 g of γ-benzyl-L-glutamate-N-carboxyanhydride was added, in an air atmosphere, to a Schlenk tube that was washed with chromic acid and had an end sealed with chlorotrimethylsilane; then 50 ml of ultra-dry 1,4-dioxane and 0.1537 g of ultra-dry triethylamine were added; a molar ratio of a monomer to the initiator triethylamine was 5:1; and then the mixture was well-mixed and allowed to react for three days at room temperature. After three days, the solution was viscous, was diluted with 1,4-dioxane, and then dropwise added to absolute ethanol to induce precipitation; and after the precipitate was filtered under vacuum and dried for 24 h, the Poly-γ-Benzyl-L-Glutamate 10 was obtained.

### 2. Preparation of poly-L-glutamic acid 12

An operation was the same as S2 in Embodiment 1, and the poly-L-glutamic acid 12 was ultimately obtained.

### Comparative example 3: Preparation of poly-L-glutamic acid 13 through magnesium hydride catalysis

### 1. Preparation of Poly-γ-Benzyl-L-Glutamate 11

2.0 g of γ-benzyl-L-glutamate-N-carboxyanhydride was added, in an air atmosphere, to a Schlenk tube that was washed with chromic acid and had an end sealed with chlorotrimethylsilane; then 50 ml of ultra-dry 1,4-dioxane and 0.0399 g of magnesium hydride were added; a molar ratio of a monomer to the magnesium hydride was 5:1; and then the mixture was well-mixed and allowed to react for three days at room temperature. After three days, the solution was viscous, was diluted with 1,4-dioxane, and then dropwise added to absolute ethanol to induce precipitation; and after the precipitate was filtered under vacuum and dried for 24 h, the Poly-γ-Benzyl-L-Glutamate 11 was obtained.

### 2. Preparation of poly-L-glutamic acid 13

An operation was the same as S2 in Embodiment 1, and the poly-L-glutamic acid 13 was ultimately obtained.

### Comparative example 4: Preparation of poly-L-glutamic acid 14 through catalysis of triethylamine containing (2%) potassium hydride

### 1. Preparation of Poly-γ-Benzyl-L-Glutamate 12

2.0 g of γ-benzyl-L-glutamate-N-carboxyanhydride was added, in an air atmosphere, to a Schlenk tube that was washed with chromic acid and had an end sealed with chlorotrimethylsilane; then 50 ml of ultra-dry 1,4-dioxane and a complex initiation system of ultra-dry triethylamine and potassium hydride (adding the potassium hydride to the triethylamine to form a premix), where the amount of the triethylamine added was 0.1537 g, and the amount of the potassium hydride added was 2% of the mass of the triethylamine; a molar ratio of a monomer to the initiator triethylamine was 5:1; and then the mixture was well-mixed and allowed to react for three days at room temperature. After three days, the solution was viscous, was diluted with 1,4-dioxane, and then dropwise added to absolute ethanol to induce precipitation; and after the precipitate was filtered under vacuum and dried for 24 h, the Poly-γ-Benzyl-L-Glutamate 12 was obtained.

### 2. Preparation of poly-L-glutamic acid 14

An operation was the same as S2 in Embodiment 1, and the poly-L-glutamic acid 14 was ultimately obtained.

### Comparative example 5: Preparation of poly-L-glutamic acid 15 through catalysis of triethylamine containing (2%) sodium hydride

### 1. Preparation of Poly-γ-Benzyl-L-Glutamate 13

2.0 g of γ-benzyl-L-glutamate-N-carboxyanhydride was added, in an air atmosphere, to a Schlenk tube that was washed with chromic acid and had an end sealed with chlorotrimethylsilane; then 50 ml of ultra-dry 1,4-dioxane and a complex initiation system of ultra-dry triethylamine and sodium hydride (adding the sodium hydride to form a premix), where the amount of the triethylamine added was 0.1537 g, and the amount of the sodium hydride added was 2% of the mass of the triethylamine; a molar ratio of a monomer to the initiator was 5:1; and then the mixture was well-mixed and allowed to react for three days at room temperature. After three days, the solution was viscous, was diluted with 1,4-dioxane, and then dropwise added to absolute ethanol to induce precipitation; and after the precipitate was filtered under vacuum and dried for 24 h, the Poly-γ-Benzyl-L-Glutamate 13 was obtained.

### 2. Preparation of poly-L-glutamic acid 15

An operation was the same as S2 in Embodiment 1, and the poly-L-glutamic acid 15 was ultimately obtained.

### Molecular weight performance detection

The polyglutamate obtained in the above embodiments and comparative examples was tested using a 0.1M LiBr DMF solution as a mobile phase, a test temperature was 45 °C, polystyrene was used as an internal standard, and a molecular weight test was performed on the polyglutamate by using GPC/SEC.

A 0.1M sodium hydrogen phosphate/sodium dihydrogen phosphate buffer solution was used as a mobile phase for the poly-L-glutamic acid 1-15, the test temperature was 45 °C, and the molecular weight test was performed on the poly-L-glutamic acid 1-15 by using a GPC/SEC triple detector.

Test results are shown in Table 1.

**Table 1: Molecular weight test for polyglutamate and poly-L-glutamic acid**

| Embodiment | Polyglutamate Mw (kDa) | Poly-L-glutamic acid Mw (kDa) | PD |
|---|---|---|---|
| Embodiment 1 | 1800 | 1671 | 1.87 |
| Embodiment 2 | 2300 | 2139 | 1.91 |
| Embodiment 3 | 2900 | 2716 | 1.69 |
| Embodiment 4 | 3900 | 3818 | 1.73 |
| Embodiment 5 | 4000 | 3927 | 1.66 |
| Embodiment 6 | 2100 | 2023 | 1.67 |
| Embodiment 7 | 2300 | 2235 | 1.59 |
| Embodiment 8 | 2500 | 2349 | 1.73 |
| Embodiment 9 | 3300 | 3178 | 1.44 |
| Embodiment 10 | 3700 | 3529 | 1.85 |
| Comparative example 1 | 800 | 712 | 1.61 |
| Comparative example 2 | 1200 | 1103 | 2.03 |
| Comparative example 3 | 700 | 631 | 1.66 |
| Comparative example 4 | 350 | 312 | 1.43 |
| Comparative example 5 | 780 | 767 | 1.92 |

Based on the above experimental data, by comparing Embodiments 1-5 with Comparative example 1, it can be seen that the molecular weight of the polymer increased with the increase in the mass fraction of the metal magnesium hydride, showing a significant rise before 20% and remaining essentially unchanged between 20% and 30%. This was because at mass fractions below 30%, the magnesium hydride might react with an initiator-derived active ion pair of the triethylamine to form a magnesium ion and NCA active intermediate ion pair, achieving a good ionic balance. As the number of ion pairs increased, a chain propagation process was accelerated, and side reactions were reduced, such that the prepared PGA had a relatively high molecular weight, reaching a plateau phase at 20%-30%. An increase in the number of ion pairs no longer dominated the course of the reaction. When the ratio exceeded 30%, the introduction of excessive magnesium ions into the system damaged an original ionic balance. The damage of the ionic balance caused free metal ions to attack NCA monomers to form excessive active ends, resulting in increased side reactions, thus leading to a reduction in the molecular weight of the obtained product.

Through Embodiments 6-7 and Comparative examples 4-5, it can be seen that, with the same content (2%) of the metal hydride, the molecular weight of the PGA product prepared in the embodiments was significantly higher than that of the PGA obtained in the comparative examples. It indicated that the reaction system is selective to the metal hydride. By selecting hydrides of highly-electronegative metal ions such as calcium and copper, these metal ions might form the loose ion pairs with the NCA active intermediates, promoting chain propagation, thereby obtaining the PGA with a high molecular weight; and the hydrides of weakly-electronegative metal ions such as sodium and potassium formed tight ion pairs with the NCA active intermediates, not facilitating the chain growth process, thus leading to a low molecular weight of the obtained PGA.

Through Embodiment 8, it can be seen that, by screening the specific metal hydride, the complex initiation system can be formed with different organic base initiators such as heterocyclic compound such as pyridine, and the glutamate NCA of different carboxyl protective agents was used as a monomer, so as to obtain the PGA with a high molecular weight (greater than 2000 kDa).

Through Embodiment 9, it can be seen that, the metal hydride can be used to promote triethylamine-initiated polymerization to form a stable loose ion pair in a non-polar organic solvent, and the monomer might be continuously added to further achieve chain growth without terminating the system, thereby increasing a molecular weight.

Through Embodiment 2 and Comparative examples 2 and 3, it can be seen that, the PGA with the high molecular weight could not be prepared in the comparative examples in which only metal hydride and triethylamine were present. This was because the magnesium hydride was in a solid state in the reaction system. It is difficult to form the initiator-derived active ion pair without the action of the triethylamine, resulting in limited chain propagation and chain growth, thus leading to a low molecular weight of the prepared PGA. In Comparative example 3, only triethylamine was present without calcium hydride, such that a loose active intermediate ion pair could not be formed, and the high-molecular weight PGA could not be prepared.

Therefore, the preparation of the ultra-high molecular weight PGA was catalyzed, on the basis of the use of organic amine as the initiator, by using the hydride corresponding to the metal ion with strong electronegativity, so as to form the complex initiation system, such that the PGA with an ultra-high molecular weight can be prepared while ensuring ionic balance.

Finally, it should be noted that, the above embodiments are merely for describing and not intended to limit the technical solutions of the present invention. Although the present invention has been described in detail with reference to the above embodiments, those of ordinary skill in the art should understand that, they can still make modifications to the technical solutions recited in the above embodiments or make equivalent replacements to a part or all of the technical features thereof; and the modifications or replacements do not cause essence of the corresponding technical solutions to depart from the scope of the technical solutions of the embodiments of the present invention.

## Claims

1. An ultra-high molecular weight Polyglutamic Acid (PGA), obtained through a ring-opening polymerization reaction that is initiated by a monomer and a complex initiation system, which is formed by a metal hydride and an initiator, wherein
the metal hydride is selected from one or more of magnesium hydride, calcium hydride, and copper hydride; and
the initiator is an organic amine compound and/or a heterocyclic compound.

2. The ultra-high molecular weight PGA according to claim 1, wherein the organic amine compound is selected from one or more of triethylamine, dimethylamine, diethylamine, dipropylamine, diisopropylamine, N-ethylmethylamine, dibutylamine, diisobutylamine, di-sec-butylamine, di-tert-butylamine, diamylamine, dioctylamine, diallylamine, dicyclohexylamine, and diphenylamine;
preferably, the heterocyclic compound is selected from one or more of pyridine, aziridine, pyrrole, pyrrolidine, imidazole, indole, piperidine, and purine
more preferably, the initiator is triethylamine or pyridine;
preferably, the monomer is glutamate-N-carboxyanhydride;
preferably, in the polymerization reaction, a molar ratio of the monomer to the initiator is (1-50):1; and/or
in the complex initiation system, the content of the metal hydride is 1-30 wt% of the initiator.

3. A method for preparing an ultra-high molecular weight Polyglutamic Acid (PGA), wherein the preparation method comprises the following steps:
(1) synthesis of polyglutamate: mixing a glutamate-N-carboxyanhydride monomer, an organic solvent A, a metal hydride, and an initiator to perform a reaction, so as to obtain the polyglutamate; and
(2) synthesis of PGA: mixing the polyglutamate obtained in step (1), an organic solvent B, and a deprotection agent to perform a reaction, so as to obtain an ultra-high molecular weight PGA, wherein
the metal hydride is selected from one or more of magnesium hydride, calcium hydride, and copper hydride.

4. The preparation method according to claim 3, wherein in step (1), the content of the metal hydride is 1-30 wt% of the initiator.

5. The preparation method according to claim 3, wherein in step (1), the glutamate-N-carboxyanhydride monomer is γ-alkyl-L-glutamate-N-carboxyanhydride or γ-aryl-L-glutamate-N-carboxyanhydride;
preferably, the alkyl is selected from methyl, ethyl, tert-butyl, allyl, or tert-butyl dimethyl; the aryl is selected from benzyl, benzyl substituted with one or more substituents selected from halogen, phenolic hydroxyl, nitro, and sulfo, benzhydryl, trityl, (ethylphenyl)methyl, naphthylmethyl, or anthrylmethyl;
more preferably, the glutamate-N-carboxyanhydride monomer is γ -benzyl-L-glutamate-N-carboxyanhydride, γ-tert-butyl-L-glutamate-N-carboxyanhydride, or γ-ethyl-L-glutamate-N-carboxyanhydride.

6. The preparation method according to claim 3, wherein in step (1), the initiator is an organic amine compound and/or a heterocyclic compound;
preferably, the organic amine compound is selected from one or more of triethylamine, dimethylamine, diethylamine, dipropylamine, diisopropylamine, N-ethylmethylamine, dibutylamine, diisobutylamine, di-sec-butylamine, di-tert-butylamine, diamylamine, dioctylamine, diallylamine, dicyclohexylamine, and diphenylamine;
preferably, the heterocyclic compound is selected from one or more of pyridine, aziridine, pyrrole, pyrrolidine, imidazole, indole, piperidine, and purine; and
more preferably, the initiator is triethylamine or pyridine.

7. The preparation method according to claim 3, wherein in step (1), a molar ratio of the glutamate-N-carboxyanhydride monomer to the initiator is (1-50):1, preferably, (5-20):1; and/or
in step (1), the reaction is performed at room temperature, and a reaction time is 1-5 days.

8. The preparation method according to claim 3, wherein in step (2), a molar ratio of the polyglutamate to the deprotection agent is 1:(1-3).

9. The preparation method according to claim 3, wherein
the organic solvent A is selected from one or more of 1,4-dioxane, hexane, cyclohexane, dichloromethane, chloroform, tetrahydrofuran, N,N-dimethylformamide, dimethyl sulfoxide, diethyl ether, diisopropyl ether, acetonitrile, methanol, ethanol, ethyl acetate, benzene, and toluene, preferably, 1,4-dioxane; and/or
the deprotection agent is a silane protective agent, and is selected from one or more of iodotrimethylsilane, trimethylsilane, tert-butyldimethylsilyl chloride, triisopropylsilyl chloride, trifluoromethanesulfonic acid tert-butyldimethylsilyl ester, and triisopropylsilyl trifluoromethanesulfonate, preferably iodotrimethylsilane; and/or
the organic solvent B is selected from one or more of dichloromethane, chloroform, tetrahydrofuran, N,N-dimethylformamide, dimethyl sulfoxide, 1,4-dioxane, hexane, cyclohexane, diethyl ether, diisopropyl ether, acetonitrile, methanol, ethanol, ethyl acetate, benzene, and toluene, preferably, dichloromethane.

10. Use of the ultra-high molecular weight Polyglutamic Acid (PGA) according to any one of claims 1 to 2 or an ultra-high molecular weight PGA prepared by the preparation method according to claims 3 to 9 in drug delivery, tissue engineering material preparation, and soft tissue repair.
